# EUROPÄISCHE PATENTSCHRIFT

(11) **EP 2 706 058 B1**
(45) Veröffentlichungstag und Bekanntmachung des Hinweises auf die Patenterteilung: **12.08.2015**
(21) Anmeldenummer: 13190284.3
(22) Anmeldetag: 11.04.2011
(51) Int. Cl.: C07D 339/08, A01N 43/32, A01P 3/00

(54) **Dithiin-Derivate als Fungizide**
Dithiin derivatives as fungicides
Dérivés de dithiine comme fungicydes

(30) Priorität: 16.04.2010 US 325030 P; 14.04.2010 EP 10159904
(43) Veröffentlichungstag der Anmeldung: 12.03.2014
(62) Teilanmeldung aus: 11713304.1
(73) Patentinhaber: Bayer Intellectual Property GmbH, 40789 Monheim (DE)
(72) Erfinder: Seitz, Thomas, 40764 Langenfeld (DE); Benting, Jürgen, 42799 Leichlingen (DE); Wachendorff-Neumann, 56566 Neuwied (DE)
(74) Vertreter: BIP Patents

(56) Entgegenhaltungen:
- WO-A1-96/19481
- WO-A1-2010/043319
- DE-B- 1 060 655
- US-A- 3 265 565
- US-A- 3 364 229
- US-A- 3 663 543
- US-A- 4 004 018
- DATABASE CAPLUS [Online] CHEMICAL ABSTRACTS SERVICE, COLUMBUS, OHIO, US; SHIGEMATSU, TAICHIRO ET AL: "1,4-Dithiaanthraquinone-2,3-dicarboximide s as fungicides", XP002719260, gefunden im STN Database accession no. 1975:542987 & JP 50 040736 B (MITSUBISHI CHEMICAL INDUSTRIES) 1975
- ZENTZ ET AL: "Syntheses, in vitro antibacterial and antifungal activities of a series of N-alkyl, 1,4-dithiines", FARMACO, SOCIETA CHIMICA ITALIANA, PAVIA, IT LNKD- DOI:10.1016/J.FARMAC.2005.06.015, Bd. 60, Nr. 11-12, 1. November 2005 (2005-11-01), Seiten 944-947, XP005151567, ISSN: 0014-827X
- GOKER H ET AL: "Synthesis and potent antifungal activity against Candida species of some novel 1H-benzimidazoles", JOURNAL OF HETEROCYCLIC CHEMISTRY 2009 HETEROCORPORATION USA LNKD- DOI:10.1002/JHET.179, Bd. 46, Nr. 5, September 2009 (2009-09), Seiten 936-948, XP002596734, ISSN: 0022-152X

## Beschreibung

Die vorliegende Erfindung betrifft die Verwendung von neuen und bekannten Dithiin-Derivaten zum Bekämpfen von unerwünschten Mikroorganismen, insbesondere phytopathogener Pilze, im Pflanzenschutz, im Bereich Haushalt und Hygiene und im Materialschutz, sowie neue Dithiin-Derivate, Verfahren zu deren Herstellung, deren Verwendung sowie Pflanzenschutzmittel enthaltend diese neuen Dithiin-Derivate.

Verschiedene Dithiin-Derivate oder auch Dihydro-dithiine sind bereits als Fungizide oder Insektizide bekannt (vgl. u.a. WO 95/29181, US 4,150,130, JP-B 48-11020, JP-A 50-40736, JP-B 52-31407, WO 2010/043319, DE-B 1060655, US 3,663,543, US 3,265,565, US 4,004,018). Andere Dithiin-Derivate mit antibakterieller Wirkung sind ebenfalls bekannt (vgl. Il Farmaco 2005, 60, 944-947, WO 96/19481). Weitere Dithiin-Derivate sind als Chemikalien, z.B. als Katalysatoren, bekannt (vgl. DE-C 362986, US 4,265,807, US 4,067,912, Chem. News and J. Phys. Sci. 1890, 62, 216-217, J. Am. Chem. Soc. 1953, 75, 2065-2069). 1,4-Dithiaanthraquinon-2,3-dicarboximide sind ebenfalls als Fungizide beschrieben (vgl. XP002719260 und JP 50 040736 A).

Da sich die ökologischen und ökonomischen Anforderungen an moderne Fungizide laufend erhöhen, beispielsweise was Wirkungsspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Fungizide zu entwickeln, die zumindest in Teilbereichen die genannten Anforderungen besser erfüllen.

Die vorliegende Erfindung betrifft nun die Verwendung von Dithiin-Derivaten der Formeln (I) 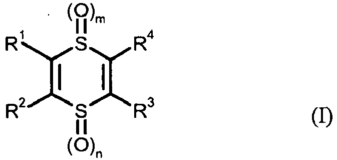 in welcher
(c)
   - m und n: jeweils für 0 stehen,
   - R¹ und R²: zusammen für die Gruppe -C(=O)-X¹-C(=O)- stehen,
   - R³ und R⁴: zusammen für die Gruppe -C(=O)-X²-C(=O)- stehen,
   - X¹: für S oder NR7 steht,
   - X²: für S oder NR7a steht, wobei X2 gleich oder verschieden von X1 ist, oder für Phenyl-1,2-diyl steht,
   - R⁷: für C₁-C₈-Alkylthio, C₁-C₈-Halogenalkylthio, C₁-C₄-Alkylamino, Di-(C₁-C₄-alkyl)amino, Phenylsulfonylamino oder für dreifach, gleich oder verschieden, durch Halogen, C₁-C₄-Alkyl, C₁-C₄-Halogenalkyl, C₁-C₄-Alkoxy, C₁-C₄-Halogenalkoxy, C₁-C₈-Alkylthio, C₁-C8-Halogenalkylthio substituiertes Aryl oder Aryl-(C₁-C₄-alkyl) steht;
   - R^{7a}: für Wasserstoff, C₁-C₈-Alkytthio, C₁-C₈-Halogenalkylthio, C₁-C₄-Alkylamino, Di-(C₁-C4-alkyl)amino, Phenylsulfonylamino oder für dreifach, gleich oder verschieden, durch Halogen, C₁-C₄-Alkyl, C₁-C₄-Halogenalkyl, C₁-C₄-Alkoxy, C₁-C₄-Halogenalkoxy, C₁-C₈-Alkylthio, C₁-C₈-Halogenalkylthio substituiertes Aryl oder Aryl-(C₁-C₄-alkyl) steht;
zum Bekämpfen von unerwünschten Mikroorganismen, insbesondere phytopathogener Pilze, sowohl im Pflanzenschutz, im Bereich Haushalt und Hygiene als auch im Materialschutz.

Die erfindungsgemäßen Dithiin-Derivate sind durch die Formel (I) allgemein definiert. Bevorzugt verwendbare Dithiin-Derivate der Formel (I) sind solche, in welcher die Reste die nachfolgenden Bedeutungen haben. Diese bevorzugten Bedeutungen gelten für die Zwischenprodukte bei der Herstellung von Verbindungen der Formel (I) in gleicher Weise.

Eine Ausführungsform (Gruppe A) der erfindungsgemäß verwendbaren Dithiin-Derivate ist durch die Formel (I) beschrieben, wobei R1, R2, R3 und R4 die Bedeutungen wie oben unter (a) genannt haben. Insbesondere haben die Dithiin-Derivate in dieser Ausführungsform keine anellierten Ringe.

Eine weitere Ausführungsform (Gruppe C) der erfindungsgemäßen Dithiin-Derivate kann durch die Formel (I-b) beschrieben werden: 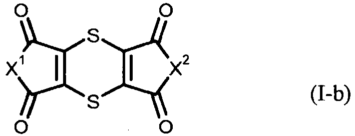 in welcher X1 und X2 die oben angegebenen Bedeutungen haben.
- X¹: steht bevorzugt für S.
- X¹: steht auch bevorzugt für NR⁷.
- X²: steht bevorzugt für S.
- X²: steht auch bevorzugt für NR7a, wobei X2 gleich oder verschieden von X1.
- X²: steht auch bevorzugt für Phenyl-1,2-diyl.
- R⁷: steht bevorzugt für C₁-C₆-Alkylthio, C₁-C₆-Halogenalkylthio, C₁-C₃-Alkylamino, Di-(C₁-C₃-alkyl)amino, Phenylsulfonylamino oder für dreifach, gleich oder verschieden, durch Fluor, Chlor, Brom, Methyl, Trifluormethyl, Methoxy, Trifluormethoxy, Methylthio, Trifluormethylthio, substituiertes Phenyl oder Phenyl-(C1-C4-alkyl).
- R⁷: steht besonders bevorzugt für Methylthio, Ethylthio, Trifluormethylthio, Trichlormethylthio, Methylamino, Dimethylamino, Phenylsulfonylamino, oder für dreifach, gleich oder verschieden, durch Fluor, Chlor, Brom, Methyl, Trifluormethyl, Methoxy, Trifluormethoxy, Methylthio, Trifluormethylthio, substituiertes Phenyl.
- R^{7a}: steht bevorzugt für Wasserstoff, C₁-C₆-Alkylthio, C₁-C₆-Halogenalkylthio, C₁-C₃-Alkylamino, Di-(C1-C3-alkyl)amino, Phenylsulfonylamino oder für dreifach, gleich oder verschieden, durch Fluor, Chlor, Brom, Methyl, Trifluormethyl, Methoxy, Trifluormethoxy, Methylthio, Trifluormethylthio, substituiertes Phenyl oder Phenyl-(C1-C4-alkyl).
- R^{7a}: steht besonders bevorzugt für Wasserstoff, Methylthio, Ethylthio, Trifluormethylthio, Trichlormethylthio, Methylamino, Dimethylamino, Phenylsulfonylamino, oder für dreifach, gleich oder verschieden, durch Fluor, Chlor, Brom, Methyl, Trifluormethyl, Methoxy, Trifluormethoxy, Methylthio, Trifluormethylthio, substituiertes Phenyl.

Im Einzelnen sei auf die in den Herstellungsbeispielen genannten Verbindungen verwiesen.

Die erfindungsgemäß verwendbaren Dithiin-Derivate können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Dithün-Derivate der Formel (I-b) (Gruppe C) lassen sich analog bekannter Methoden herstellen (vgl. US 3,364,229, Synthetic Commun. 2006, 36, 3591-3597, Il Farmaco 2005, 60, 944-947 und WO 2010/043319). Die vorliegende Erfindung betrifft weiterhin ein Pflanzenschutzmittel zum Bekämpfen unerwünschter Pilze umfassend wenigstens eines der Dithiin-Derivate der Formel (I). Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Außerdem betrifft die Erfindung ein Verfahren zum Bekämpfen unerwünschter Mikroorganismen, dadurch gekennzeichnet, dass man erfindungsgemäß Dithiin-Derivate der Formel (I) auf die phytopathogenen Pilze und/oder deren Lebensraum ausbringt.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.

Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobemsteinsäureestem, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl-dispergierbare Pulver, Öl-mischbare fließfähige Konzentrate, Öl-mischbare Flüssigkeiten, Schäume, Pasten, Pestizid-ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgutbehandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die Erfindung umfasst weiterhin ein Verfahren zur Behandlung von Saatgut.

Die Erfindung betrifft weiterhin Saatgut, welches gemäß einem der im vorherigen Absatz beschriebenen Verfahren behandelt wurde. Die erfindungsgemäßen Saatgüter finden Anwendung in Verfahren zum Schutz von Saatgut vor unerwünschten Pilzen. Bei diesen wird ein mit wenigstens einem erfindungsgemäßen Wirkstoff behandeltes Saatgut verwendet.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt werden, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von phytopathogenen Pilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von phytopathogenen Pilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Wirkstoffe bzw. Mittel die Behandlung des Saatguts mit diesen Wirkstoffen bzw. Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Garten- und Weinbau eingesetzt wird. Insbesondere handelt es sich dabei um Saatgut von Getreide (wie Weizen, Gerste, Roggen, Triticale, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Raps, Mohn, Olive, Kokosnuss, Kakao, Zuckerrohr, Tabak, Gemüse (wie Tomate, Gurke, Zwiebeln und Salat), Rasen und Zierpflanzen (siehe auch unten). Besondere Bedeutung kommt der Behandlung des Saatguts von Getreide (wie Weizen, Gerste, Roggen, Triticale und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European com borer) und/oder Western Corn Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutylnaphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Wirkstoffe bzw. Mittel weisen eine starke fungizide Wirkung auf und können zur Bekämpfung von unerwünschten Pilzen im Pflanzenschutz und im Materialschutz eingesetzt werden.

Die erfindungsgemäßen Dithiin-Derivate lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht werden.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp*., *Betulaceae sp., Anacardiaceae sp., Fagaceae sp., Moraceae sp., Oleaceae sp., Actinidaceae sp*., Lauraceae *sp*., *Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnubpflanzen, wie *Gramineae sp.* (beispielsweise Mais, Rasen, Getreide wie Weizen, Roggen, Reis, Gerste, Hafer, Hirse und Triticale), *Poaceae sp.* (z.B. Zuckerrohr), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, dass die behandelten Pflanzen, wenn sie im Anschluss daran mit unerwünschten phytopathogenen Pilzen inokuliert wurde, einen beträchtlichen Resistenzgrad gegen diese unerwünschten phytopathogenen Pilze aufweisen. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Stressfaktoren resistent, d.h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Stressfaktoren resistent sind. Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Stress- und Nicht-StressBedingungen) beeinflusst werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium*, das CP4-Gen des Bakteriums *Agrobacterium sp*., die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, dass man ein Gen exprimiert, das für ein Glyphosate-Oxidoreduktase-Enzym kodiert. Glyphosatetolerante Pflanzen können auch dadurch erhalten werden, dass man ein Gen exprimiert, das für ein Glyphosate-acetyltransferase-Enzym kodiert. Glyphosatetolerante Pflanzen können auch dadurch erhalten werden, dass man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmem tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylhamstoff- und imidazolinontolerante Pflanzen sind auch in z.B. WO 2007/024782 beschrieben.

Weitere Pflanzen, die gegenüber Imidazolinon und/oder Sulfonylhamstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfasst im vorliegenden Zusammenhang jegliche Pflanze, die mindestens ein Transgen enthält, das eine Kodiersequenz umfasst, die für folgendes kodiert:
1) ein insektizides Kristallprotein aus *Bacillus thuringiensis* oder einen insektiziden Teil davon, wie die insektiziden Kristallproteine, die online bei:
   http://www.lifesci.sussex.ac.uk/Home/Neil_Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfasst, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfasst, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfasst, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein stresstoleranzförderndes Transgen enthalten, das die Expression und/oder Aktivität der für PARG kodierenden Gene der Pflanzen oder Pflanzenzellen zu reduzieren vermag;
c. Pflanzen, die ein stresstoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylhamstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die erfindungsgemäßen Wirkstoffe bzw. Mittel können außerdem im Materialschutz zum Schutz von technischen Materialien gegen Befall und Zerstörung durch unerwünschte Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor pilzlicher Veränderung oder Zerstörung geschützt werden sollen, Klebstoffe, Leime, Papier, Wandpappe und Karton, Textilien, Teppiche, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen und Gebäuden, z.B. Kühlwasserkreisläufe, Kühl- und Heizsysteme und Belüftungs- und Klimaanlagen, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern. Außerdem können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Das erfindungsgemäße Verfahren zum Bekämpfen von unerwünschten Pilzen kann auch zum Schutz von so genannten Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus;
Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola; Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici; Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa; Deformationen von Blättern, Blüten und Früchten, hervorgerufen durch z.B. Taphrina-Arten, wie beispielsweise Taphrina deformans;
Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora.

Bevorzugt können die folgenden Krankheiten von Soja-Bohnen bekämpft werden:
Pilzkrankheiten an Blättern, Stängeln, Schoten und Samen verursacht durch z.B. Alternaria leaf spot (Altemaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).
Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Als Organismen, die einen Abbau oder eine Veränderung der technischen Materialien bewirken können, seien Pilze genannt. Vorzugsweise wirken die erfindungsgemäßen Wirkstoffe gegen Pilze, insbesondere Schimmelpilze, Holz verfärbende und Holz zerstörende Pilze (Basidiomyceten). Es seien beispielsweise Pilze der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride.

Darüber hinaus weisen die erfindungsgemäßen Wirkstoffe auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Die aufgefiihrten Pflanzen können besonders vorteilhaft erfindungsgemäß mit den Dithiin-Derivaten der Formel (I) oder den erfindurigsgemäßen Mitteln behandelt werden. Die bei den Wirkstoffen bzw. Mitteln oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mitteln.

### Herstellungsbeispiele

### Referenz-Verbindung Nr. 43

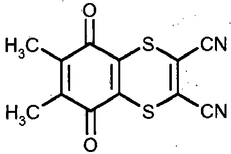

1 g (3,619 mmol) 5,8-Dihydroxy-6,7-dimethyl-1,4-benzodithiin-2,3-dicarbonitril (Verbindung Nr. 47, siehe unten) wurden unter Rückfluss in 20 mL Eisessig gelöst. In der Hitze wurden 10 mL 65%ige Salpetersäure zugegeben. Nach heißer Filtration und Abkühlung auf Raumtemperatur wurde mit 30 mL Wasser verdünnt. Die Kristalle wurden abgesaugt, mit Wasser gewaschen und an Kieselgel (Cyclohexan/Essigsäureethylester 1:1) chromatographiert. Man erhielt 223 mg (22,5 % der Theorie) an 6,7-Dimethyl-5,8-dioxo-5,8-dihydro-1,4-benzodithiin-2,3-dicarbonitril.

### Referenz-Verbindung Nr. 44

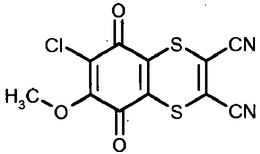

0,884 g (2,825 mmol) 6-Chlor-5,8-dihydroxy-7-methoxy-1,4-benzodithiin-2,3-dicarbonitril (Verbindung Nr. 48, siehe unten) wurden unter Rückfluss in 17 mL Eisessig gelöst. In der Hitze wurden 7 mL 65%ige Salpetersäure zugegeben. Nach heißer Filtration und Abkühlung auf Raumtemperatur wurde mit 25 mL Wasser verdünnt. Die Kristalle wurden abgesaugt und mit Wasser gewaschen. Man erhielt 415 mg (93 % Reinheit, 44,0 % der Theorie) an 6-Chlor-7-methoxy-5,8-dioxo-5,8-dihydro-1,4-benzodithiin-2,3-dicarbonitril.

### Referenz-Verbindung Nr. 45

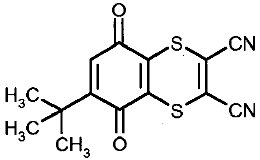

1,2 g (3,942 mmol) 6-tert-Butyl-5,8-dihydroxy-1,4-benzodithiin-2,3-dicarbonitril (Verbindung Nr. 49, siehe unten) wurden unter Rückfluss in 20 mL Eisessig gelöst. In der Hitze wurden 10 mL 65%ige Salpetersäure zugegeben. Nach heißer Filtration und Abkühlung auf Raumtemperatur wurde mit 25 mL Wasser verdünnt. Die Kristalle wurden abgesaugt, mit Wasser gewaschen und an Kieselgel (Cyclohexan/Essigsäureethylester 1:1) chromatographiert. Man erhielt 346 mg (29,0 % der Theorie) an 6-tert-Butyl5,8-dioxo-5,8-dihydro-1,4-benzodithiin- 2,3-dicarbonitril.

### Referenz-Verbindung Nr. 47

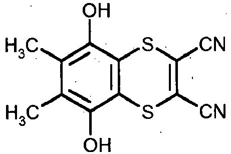

2,32 g (11,315 mmol) 2,3-Dichlor-5,6-dimethyl-1,4-benzochinon wurden in 23 mL N,N-Dimethylformamid und 45 mL Essigsäure vorgelegt. Unter Eiskühlung wurden 4,621 g (22,630 mmol) Dinatrium-1,2-dicyanethen-1,2-bis(thiolat)-Hydrat, gelöst in 23 mL Wasser, tropfenweise zugegeben und 30 Minuten unter Eiskühlung nachgerührt. Nach Zugabe von weiteren 23 mL Wasser wurde ein rötlicher Feststoff abfiltriert, mit Wasser nachgewaschen und getrocknet. Man erhielt 2,30 g (97 % Reinheit, 71,4 % der Theorie) an 5,8-Dihydroxy-6,7-dimethyl-1,4-benzodithiin-2,3-dicarbonitril.

### Referenz-Verbindung Nr. 48

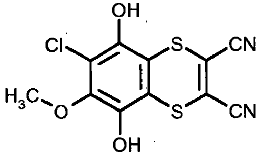

1 g (4,142 mmol) 2,3,5-trichlor-6-methoxy-1,4-benzochinon wurden in 10 mL N,N-Dimethylformamid und 20 mL Essigsäure vorgelegt. Unter Eiskühlung wurden 1,691 g (8,283 mmol) Dinatrium-1,2-dicyanethen-1,2-bis(thiolat)-Hydrat, gelöst in 23 mL Wasser, tropfenweise zugegeben und 30 Minuten unter Eiskühlung nachgerührt. Nach Zugabe von weiteren 10 mL Wasser wurde ein rötlicher Feststoff abfiltriert, mit Wasser nachgewaschen und getrocknet. Man erhielt 934 mg (90 % Reinheit, 64,9 % der Theorie) an 6-Chlor-5,8-dihydroxy-7-methoxy-1,4-benzodithiin-2,3-dicarbonitril.

### Referenz-Verbindung Nr. 49

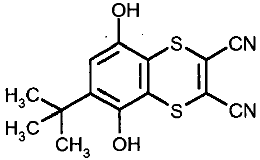

1 g (4,29 mmol) 5-tert-butyl-2,3-dichlor-1,4-benzochinon wurden in 10 mL N,N-Dimethylformamid und 20 mL Essigsäure vorgelegt. Unter Eiskühlung wurden 1,752 g (8,58 mmol) Dinatrium-1,2-dicyanethen-1,2-bis(thiolat)-Hydrat, gelöst in 23 mL Wasser, tropfenweise zugegeben und 30 Minuten unter Eiskühlung nachgerührt. Nach Zugabe von weiteren 10 mL Wasser wurde mit Methylenchlorid extrahiert, getrocknet und eingeengt. Man erhielt 1,30 mg (90 % Reinheit, 89,59 % der Theorie) an 6-tert-Butyl-5,8dihydroxy-1,4-benzodithiin-2,3-dicarbonitril als braunes Öl.

Analog den vorangehenden Beispielen sowie entsprechend den allgemeinen Beschreibungen der erfindungsgemäßen Verfahren können die in den folgenden Tabellen genannten Verbindungen der Formel (I) erhalten werden.

**Tabelle 1: Referenz-Beispiele**

| | | | | | | | |
|---|---|---|---|---|---|---|---|
| 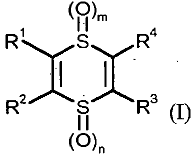 | | | | | | | |

| **Nr.** | **m** | **n** | **R¹** | **R²** | **R³** | **R⁴** | **Log P** |
|---|---|---|---|---|---|---|---|
| 1 | 0 | 0 | Ph | N-Phenylmörpholin-4-carboximidoyl | Ph | N-Phenylmorpholin-4-carboximidoyl | |
| 2 | 0 | 0 | Br | Ph | Br | Ph | |
| 3 | 0 | 0 | H | 4-NO₂-Ph | H | 4-NO₂-Ph | |
| 4 | 0 | 0 | H | 4-MeO-Ph | H | 4-MeO-Ph | |
| 5 | 0 | 0 | CO₂Me | CO₂M | CO₂Me | CO₂Me | |
| 6 | 0 | 0 | H | Ph | H | Ph | |
| 7 | 0 | 0 | NO₂ | Ph | H | Ph | |
| 8 | 0 | 0 | NO₂ | Ph | NO₂ | Ph | |
| 9 | 0 | 0 | H | 2,4-Cl₂-Ph | H | 2,4-Cl₂-Ph | |
| 10 | 0 | 0 | H | 2-Cl-Ph | H | 2-Cl-Ph | |
| 11 | 0 | 0 | Ph | Ph | Ph | Ph | |
| 12 | 0 | 0 | Me | OEt | Me | OEt | |
| 13 | 0 | 0 | H | CH₂CO₂Et | H | CH₂CO₂Et | |
| 14 | 0 | 0 | NO₂ | Ph | Br | Ph | |
| 15 | 2 | 0 | H | Ph | Br | Ph | |
| 16 | 0 | 0 | H | 4-MeO-Ph | H | Ph | |
| 17 | 0 | 0 | H | Ph | Ph | H | |
| 18 | 2 | 2 | H | Ph | Ph | H | |
| 19 | 0 | 0 | H | 1,3-Thiazol-4-yl | H | 1,3-Thiazol-4-yl | |
| 20 | 0 | 0 | H | 4-Me-Ph | H | 4-Me-Ph | |
| 21 | 0 | 0 | H | 4-F-Ph | H | 4-F-Ph | |
| 22 | 0 | 0 | H | 4-Ph-Ph | H | 4-Ph-Ph | |
| 23 | 0 | 0 | H | 2-Nph | H | 2-Nph | |
| 24 | 0 | 0 | CO₂H | CO₂H | CO₂H | CO₂H | |
| 25 | 0 | 0 | CO₂H | CO₂Me | CO₂H | CO₂Me | |
| 26 | 0 | 0 | CO₂Me | 1-Nph | CO₂Me | 1-Nph | |

| | | | | | | | |
|---|---|---|---|---|---|---|---|
| Me = Methyl, Et = Ethyl, Ph = Phenyl, Nph = Naphthyl | | | | | | | |

**Tabelle 2: Referenz-Beispiele**

| | | | | | |
|---|---|---|---|---|---|
| 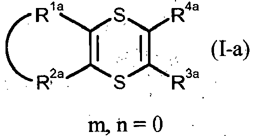 | | | | | |

| **Nr.** | **R^{1a}** | **R^{2a}** | **R^{3a}** | **R^{4a}** | **Log P** |
|---|---|---|---|---|---|
| 27 | -N=C(NMe₂)-S- | | -S-C(NMe₂)=N- | | |
| 28 | -S-C(=S)-S- | | 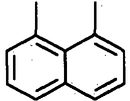 | | |
| 29 | -N(tBu)-(CH₂)₂- | | -(CH₂)₂-N(tBu)- | | |
| 30 | -C(=O)-N(Me)-C(=O)-N(Me)- | | -C(=O)-N(Me)-C(=O)-N(Me)- | | |
| 31 | -CH=CH-S- | | =CH=CH-S- | | |
| 32 | -CH=C(COMe)-S- | | -CH=CH-S- | | |
| 33 | -CH=C(COMe)-S- | | -S-CH=CH- | | |
| 34 | 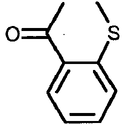 | | 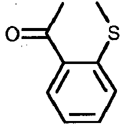 | | |
| 35 | -S-C(=S)-S- | | H | H | |

| | | | | | |
|---|---|---|---|---|---|
| tBu = tert-Butyl | | | | | |

**Tabelle 3: erfindungsgemäße Verbindungen**

| | | | |
|---|---|---|---|
| 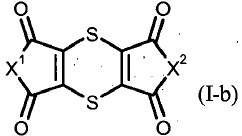 | | | |

| **Nr.** | **X¹** | **X²** | **Log P** |
|---|---|---|---|
| 36 | S | S | |
| 37 | N-(4-Cl-2-F-5-OMe-Ph) | N-(4-Cl-2-F-5-OMe-Ph) | |
| 38 | N-Ph | Phenyl-1,2-diyl | |
| 39 | N-NH-SO₂-Ph | N-NH-SO₂-Ph | |
| 40 | N-NMe₂ | N-NMe₂ | |
| 41 | NH | N-SCCl₃ | |

| | | | |
|---|---|---|---|
| Me = Methyl, Ph = Phenyl | | | |

**Tabelle 4: Referenz-Beispiele**

| | | | | | |
|---|---|---|---|---|---|
| 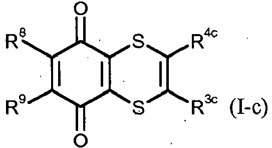 | | | | | |

| **Nr.** | **R⁸** | **R⁹** | **R^{3c}** | **R^{4c}** | **Log P** |
|---|---|---|---|---|---|
| 42 | -CH=CH-CH=CH- | | 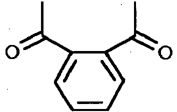 | | |
| 43 | Me | Me | CN | CN | |
| 44 | Cl | OMe | CN | CN | |
| 45 | H | tBu | CN | CN | |

| | | | | | |
|---|---|---|---|---|---|
| CN = Cyano, Me = Methyl, tBu = tert-Butyl | | | | | |

**Tabelle 5: Referenz-Beispiele**

| | | | | | |
|---|---|---|---|---|---|
| 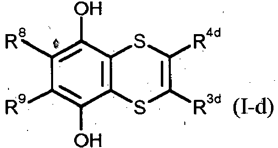 | | | | | |

| **Nr.** | **R⁸** | **R⁹** | **R^{3d}** | **R^{4d}** | **Log P** |
|---|---|---|---|---|---|
| 46 | Me | Me | -C(=O)-C(Me)=C(Me)-C(=O)- | | |
| 47 | Me | Me | CN | CN | 2,26 |
| 48 | Cl | OMe | CN | CN | 2,19 |
| 49 | H | tBu | CN | CN | 2,95 |

| | | | | | |
|---|---|---|---|---|---|
| CN = Cyano, Me = Methyl, tBu = tert-Butyl | | | | | |

Die Bestimmung der in den voran stehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel C: Phytophthora-Test (Tomate) / protektiv

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit werden junge Tomatenpflanzen mit der Wirkstoffzubereitung in der angegebenen Aufwandmenge besprüht. 1 Tag nach der Behandlung werden die Pflanzen mit einer Sporensuspension von *Phytophthora infestans* inokuliert und stehen dann 24 h bei 100 % rel. Feuchte und 22°C. Anschließend werden die Pflanzen in einer Klimazelle bei ca. 96 % relativer Luftfeuchtigkeit und einer Temperatur von ca. 20°C aufgestellt. 7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird. In diesem Test zeigen die nachfolgenden erfindungsgemäßen Verbindungen bei einer Konzentration an Wirkstoff von 1500 ppm einen Wirkungsgrad von 70 % oder mehr.

**Tabelle C: Phytophthora-Test (Tomate) / protektiv**

| Wirkstoff Bsp. Nr. | Strukturtyp | Aufwandmenge in ppm | Wirkungsgrad in % |
|---|---|---|---|
| 40 | (I-b) | 1500 | 75 |
| 41 | (I-b) | 1500 | 89 |

## Patentansprüche

1. Verwendung von Dithiin-Derivaten der Formel (I) 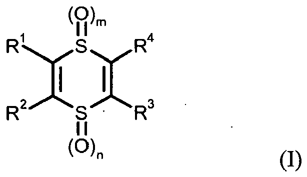 in welcher
(c)
m und n jeweils für 0 stehen,
R1 und R2 zusammen für die Gruppe -C(=O)-X1-C(=O)- stehen,
R3 und R4 zusammen für die Gruppe -C(=O)-X2-C(=O)- stehen,
X1 für S oder NR7 steht,
X2 für S oder NR7a steht, wobei X2 gleich oder verschieden von X1 ist, oder für Phenyl-1,2-diyl steht,
R7 für C1-C8-Alkylthio, C1-C8-Halogenalkylthio, C1-C4-Alkylamino, Di-(C1-C4-alkyl)amino, Phenylsulfonylamino oder für dreifach, gleich oder verschieden, durch Halogen, C1-C4-Alkyl, C1-C4-Halogenalkyl, C1-C4-Alkoxy, C1-C4-Halogenalkoxy, C1-C8-Alkylthio, C1-C8-Halogenalkylthio substituiertes Aryl oder Aryl-(C1-C4-alkyl) steht;
R7a für Wasserstoff, C1-C8-Alkylthio, C1-C8-Halogenalkylthio, C1-C4-Alkylamino, Di-(C1-C4-alkyl)amino, Phenylsulfonylamino oder für dreifach, gleich oder verschieden, durch Halogen, C1-C4-Alkyl, C1-C4-Halogenalkyl, C1-C4-Alkoxy, C1-C4-Halogenalkoxy, C1-C8-Alkylthio, C1-C8-Halogenalkylthio substituiertes Aryl oder Aryl-(C1-C4-alkyl) steht;
zum Bekämpfen von unerwünschten Mikroorganismen, insbesondere phytopathogener Pilze, sowohl im Pflanzenschutz, im Bereich Haushalt und Hygiene als auch im Materialschutz.

2. Dithiin-Derivate der Formel (I-b) 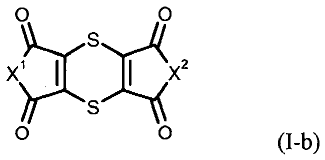
X1 für S oder NR7 steht,
X2 für S oder NR7a steht, wobei X2 gleich oder verschieden von X1 ist, oder für Phenyl-1,2-diyl steht,
R7 für C1-C8-Alkylthio, C1-C8-Halogenalkylthio, C1-C4-Alkylamino, Di-(C1-C4-alkyl)amino, Phenylsulfonylamino oder für dreifach, gleich oder verschieden, durch Halogen, C1-C4-Alkyl, C1-C4-Halogenalkyl, C1-C4-Alkoxy, C1-C4-Halogenalkoxy, C1-C8-Alkylthio, C1-C8-Halogenalkylthio substituiertes Aryl oder Aryl-(C1-C4-alkyl) steht;
R7a für Wasserstoff, C1-C8-Alkylthio, C1-C8-Halogenalkylthio, C1-C4-Alkylamino, Di-(C1-C4-alkyl)amino, Phenylsulfonylamino oder für dreifach, gleich oder verschieden, durch Halogen, C1-C4-Alkyl, C1-C4-Halogenalkyl, C1-C4-Alkoxy, C1-C4-Halogenalkoxy, C1-C8-Alkylthio, C1-C8-Halogenalkylthio substituiertes Aryl oder Aryl-(C1-C4-alkyl) steht.

3. Verwendung von Dithiin-Derivaten der Formel (I) gemäß Anspruch 1 zum Bekämpfen phytopathogener Pilze im Pflanzenschutz und Materialschutz.

4. Verfahren zum Bekämpfen unerwünschter Mikroorganismen, **dadurch gekennzeichnet, dass** man Dithiin-Derivate der Formel (I) gemäß Anspruch 1 auf die Mikroorganismen und/oder deren Lebensraum ausbringt.

5. Verfahren zum Herstellen von Mitteln zum Bekämpfen unerwünschter Mikroorganismen, **dadurch gekennzeichnet, dass** man Dithiin-Derivate der Formel (I) gemäß Anspruch 1 mit Streckmitteln und/oder oberflächenaktiven Stoffen vermischt.

6. Verwendung von Dithiin-Derivate der Formel (I) gemäß Anspruch 1 zur Behandlung von transgenen Pflanzen.

7. Mittel enthaltend mindestens eines der Dithiin-Derivate der Formel (I) gemäß Anspruch 1 sowie mindestens einen anderen Wirkstoff ausgewählt aus der Gruppe der Insektizide, Lockstoffe, Sterilantien, Bakterizide, Akarizide, Nematizide, Fungizide, Wachstumsregulatoren, Herbizide, Düngemittel, Safener und Semiochemicals.

## Claims

1. Use of dithiine derivatives of the formula (I) 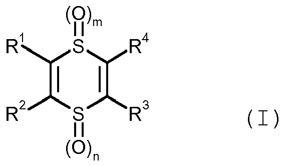 in which (c)
m and n are each 0,
R^{I} and R² together are the group -C (=O)-X¹-C(=O)-,
R³ and R⁴ together are the group -C(=O)-X²-C (=O)-,
X¹ is S or NR⁷,
X² is S or NR^{7a}, and X² is identical to or different from X¹, or is phenyl-1,2-diyl,
R⁷ is C₁-C₈-alkylthio, C₁-C₈-haloalkylthio, C₁-C₄-alkylamino, di (C₁-C₄-alkyl)amino or phenylsulphonylamino or is aryl or aryl-(C₁-C₄-alkyl) substituted three times by identical or different halogen, C₁-C₄-alkyl, C₁-C₄-haloalkyl, C₁-C₄-alkoxy, C₁-C₄-haloalkoxy, C₁-C₈-alkylthio or C₁-C₈-haloalkylthio substituents;
R^{7a} is hydrogen, C₁-C₈-alkylthio, C₁-C₈-haloalkylthio, C₁-C₄-alkylamino, di (C₁-C₄-alkyl)amino or phenylsulphonylamino or is aryl or aryl- (C₁-C₄-alkyl) substituted three times by identical or different halogen, C₁-C₄-alkyl, C₁-C₄-haloalkyl, C₁-C₄-alkoxy, C₁-C₄-haloalkoxy, C₁-C₈-alkyl thio or C₁-C₈-haloalkylthio substituents;
for controlling unwanted microorganisms, more particularly phytopathogenic fungi, in crop protection, in the household and hygiene sector, and in the protection of materials.

2. Dithiine derivatives of the formula (I-b) 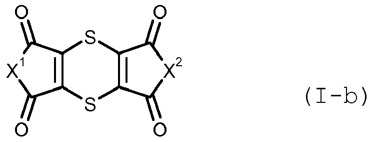 in which
X¹ is S or NR⁷,
X² is S or NR^{7a}, and X² is identical to or different from X¹, or is phenyl-1,2-diyl,
R⁷ is C₁-C₈-alkylthio, C₁-C₈-haloalkylthio, C₁-C₄-alkylamino, di (C₁-C₄-alkyl)amino or phenylsulphonylamino or is aryl or aryl-(C₁-C₄-alkyl) substituted three times by identical or different halogen, C₁-C₄-alkyl, C₁-C₄-haloalkyl, C₁-C₄-alkoxy, C₁-C₄-haloalkoxy, C₁-C₈-alkylthio or C₁-C₈-haloalkylthio substituents;
R^{7a} is hydrogen, C₁-C₈-alkylthio, C₁-C₈-haloalkylthio, C₁-C₄-alkylamino, di (C₁-C₄-alkyl)amino or phenylsulphonylamino or is aryl or aryl- (C₁-C₄-alkyl) substituted three times by identical or different halogen, C₁-C₄-alkyl, C₁-C₄-haloalkyl, C₁-C₄-alkoxy, C₁-C₄-haloalkoxy, C₁-C₈-alkylthio or C₁-C₈-haloalkylthio substituents.

3. Use of dithiine derivatives of the formula (I) according to Claim 1 for controlling phytopathogenic fungi in crop protection and the protection of materials.

4. Method for controlling unwanted microorganisms, **characterized in that** dithiine derivatives of the formula (I) according to Claim 1 are delivered to the microorganisms and/or their habitat.

5. Process for producing compositions for controlling unwanted microorganisms, **characterized in that** dithiine derivatives of the formula (I) according to Claim 1 are mixed with extenders and/or surface-active substances.

6. Use of dithiine derivatives of the formula (I) according to Claim 1 for treating transgenic plants.

7. Compositions comprising at least one of the dithiine derivatives of the formula (I) according to Claim 1 and also at least one other active compound selected from the group consisting of insecticides, attractants, sterilants, bactericides, acaricides, nematicides, fungicides, growth regulators, herbicides, fertilizers, safeners and semiochemicals.

## Revendications

1. Utilisation de dérivés de dithiine de formule (I) 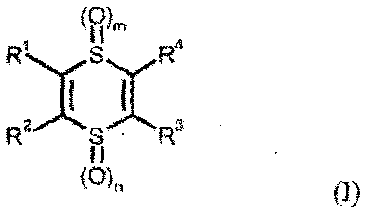 dans laquelle
(c) m et n représentent chacun 0,
R1 et R2 représentent ensemble le groupe -C (=O)-X1-C(=O)-,
R3 et R4 représentent ensemble le groupe -C(=O)-X2-C(=O)-,
X1 représente S ou NR7,
X2 représente S ou NR7a, X2 étant identique ou différent de X1, ou représente phényl-1,2-diyle,
R7 représente alkylthio en C1-C8, halogénoalkylthio en C1-C8, alkylamino en C1-C4, di-(alkyle en C1-C4)amino, phénylsulfonylamino, ou aryle ou aryl-(alkyle en C1-C4) substitué trois fois, de manière identique ou différente, par halogène, alkyle en C1-C4, halogénoalkyle en C1-C4, alcoxy en C1-C4, halogénoalcoxy en C1-C4, alkylthio en C1-C8, halogénoalkylthio en C1-C8 ;
R7a représente hydrogène, alkylthio en C1-C8, halogénoalkylthio en C1-C8, alkylamino en C1-C4, di-(alkyle en C1-C4)amino, phénylsulfonylamino, ou aryle ou aryl-(alkyle en C1-C4) substitué trois fois, de manière identique ou différente, par halogène, alkyle en C1-C4, halogénoalkyle en C1-C4, alcoxy en C1-C4, halogénoalcoxy en C1-C4, alkylthio en C1-C8, halogénoalkylthio en C1-C8 ;
pour lutter contre les microorganismes indésirables, notamment les champignons phytopathogènes, dans la protection des plantes, dans le domaine du ménage et de l'hygiène et dans la protection des matériaux.

2. Dérivés de dithiine de formule (I-b) 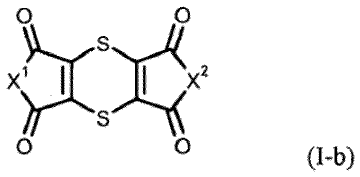 dans laquelle
X1 représente S ou NR7,
X2 représente S ou NR7a, X2 étant identique ou différent de X1, ou représente phényl-1,2-diyle,
R7 représente alkylthio en C1-C8, halogénoalkylthio en C1-C8, alkylamino en C1-C4, di-(alkyle en C1-C4)amino, phénylsulfonylamino, ou aryle ou aryl-(alkyle en C1-C4) substitué trois fois, de manière identique ou différente, par halogène, alkyle en C1-C4, halogénoalkyle en C1-C4, alcoxy en C1-C4, halogénoalcoxy en C1-C4, alkylthio en C1-C8, halogénoalkylthio en C1-C8 ;
R7a représente hydrogène, alkylthio en C1-C8, halogénoalkylthio en C1-C8, alkylamino en C1-C4, di-(alkyle en C1-C4)amino, phénylsulfonylamino, ou aryle ou aryl-(alkyle en C1-C4) substitué trois fois, de manière identique ou différente, par halogène, alkyle en C1-C4, halogénoalkyle en C1-C4, alcoxy en C1-C4, halogénoalcoxy en C1-C4, alkylthio en C1-C8, halogénoalkylthio en C1-C8.

3. Utilisation de dérivés de dithiine de formule (I) selon la revendication 1 pour lutter contre les champignons phytopathogènes dans la protection des plantes et la protection des matériaux.

4. Procédé de lutte contre les microorganismes indésirables, **caractérisé en ce que** des dérivés de dithiine de formule (I) selon la revendication 1 sont appliqués sur les microorganismes et/ou leur habitat.

5. Procédé de fabrication d'agents de lutte contre les microorganismes indésirables, **caractérisé en ce que** des dérivés de dithiine de formule (I) selon la revendication 1 sont mélangés avec des extendeurs et/ou des substances tensioactives.

6. Utilisation de dérivés de dithiine de formule (I) selon la revendication 1 pour le traitement de plantes transgéniques.

7. Agent contenant au moins un des dérivés de dithiine de formule (I) selon la revendication 1, ainsi qu'au moins un autre agent actif choisi dans le groupe constitué par les insecticides, les substances attractives, les stérilisants, les bactéricides, les acaricides, les nématicides, les fongicides, les régulateurs de croissance, les herbicides, les engrais, les agents protecteurs et les produits sémiochimiques.
